# NEUE EUROPÄISCHE PATENTSCHRIFT

(11) **EP 1 025 072 B2**
(45) Veröffentlichungstag und Bekanntmachung des Hinweises auf die Entscheidung über den Einspruch: **06.01.2010**
(45) Hinweis auf die Patenterteilung: 26.11.2003
(21) Anmeldenummer: 98952652.0
(22) Anmeldetag: 25.09.1998
(51) Int. Cl.: C07C 29/128, C07C 67/05, C07C 31/20, C07C 69/08

(54) **VERFAHREN ZUR HERSTELLUNG VON VICINALEN DIOLEN ODER POLYOLEN**
METHOD FOR PRODUCING VICINAL DIOLS OR POLYOLS
PROCEDE DE PREPARATION DE DIOLS OU DE POLYOLS VICINAUX

(30) Priorität: 29.09.1997 DE 19743015
(43) Veröffentlichungstag der Anmeldung: 09.08.2000
(73) Patentinhaber: BASF SE, 67056 Ludwigshafen (DE)
(72) Erfinder: OFTRING, Alfred, D-67098 Bad Dürkheim (DE); BOGENSTÄTTER, Thomas, D-67098 Bad Dürkheim (DE); OTT, Christian, D-67346 Speyer (DE)
(74) Vertreter: Isenbruck, Günter
(86) Internationale Anmeldenummer: PCT/EP1998/006130
(87) Internationale Veröffentlichungsnummer: WO 1999/016733

(56) Entgegenhaltungen:
- DE-A- 2 937 840
- DE-A- 3 229 084
- DE-A1- 1 944 120
- DE-B- 1 024 496
- GB-A- 2 145 076
- US-A- 2 669 572
- US-A- 2 739 173
- US-A- 4 479 021
- J. FALBE, M. REGITZ: 'Römpp Chemie Lexikon, 9. Auflage', 1993, GEORG THIEME VERLAG, STUTTGART, NEW YORK Seite 323
- 'Azeotropic Data' ADVANCES IN CHEMISTRY SERIES Nr. 6, 1952, AMERICAN CHEM. SOC., WASHINGTON D.C., US, Seite 6, 28
- J. GMEHLING ET AL: 'Azeotropic Data', Bd. 2, teil 1 2004, WILEY-VCH VERLAG GMBH, WEINHEIM, DE, ISBN 3-527-30833-4 Artikel 'S. XL-XLI, 48-51, 56, 199'
- J. FALBE, M. REGITZ: 'Römpp Chemie Lexikon', Bd. 9, 1993, GEROG THIEME VERLAG, STUTTGART, NEW YORK Artikel 'Dünnfilmverdampfer'

## Beschreibung

Die Erfindung betrifft Verfahren zur Herstellung von vicinalen Diolen oder Polyolen aus Olefinen oder Ameisensäureestern von vicinalen Diolen.

Vicinale Diole können durch Umsetzung von Olefinen mit Wasserstoffperoxid und Ameisensäure hergestellt werden. Dabei entsteht zunächst - je nach Mengenverhältnissen der Einsatzstoffe - ein Gemisch aus vicinalen Diolen und Ameisensäureestern der vicinalen Diole, wobei eine oder beide der Hydroxylgruppen verestert sein können. Um zu den vicinalen Diolen zu gelangen, müssen die Ameisensäureester gespalten werden. Hierzu ist eine Reihe von Verfahren bekannt.

In der DE-A-32 29 084 ist ein Verfahren zur Herstellung von vicinalen Diolen und deren Formiaten beschrieben, wobei zunächst Olefine mit Ameisensäure und Wasserstoffperoxid umgesetzt werden, und das Umsetzungsprodukt mit katalytischen Mengen an Alkali- oder Erdalkalimetallalkoholaten zur Spaltung der Ester umgesetzt wird.

Die DE-A-29 37 840 betrifft ein Verfahren zur Hydroxylierung von kürzerkettigen aliphatischen Mono- oder Diolefinen. Die Mono- oder Diolefine werden mit Ameisensäure und Wasserstoffperoxid umgesetzt, und das Reaktionsgemisch wird nachfolgend mit Natronlauge neutralisiert und mit Ethylacetat extrahiert, um zu den gewünschten Diolen zu gelangen.

US 4,479,021 betrifft ein kontinuierliches Verfahren zur Herstellung von 1,2-Alkandiolen. Dazu werden 1,2-Olefine mit Ameisensäure und Wasserstoffperoxid umgesetzt, worauf das erhaltene Alkandiolmonoformiat in einem mehrstufigen Verfahren verseift wird. Die Verseifung wird durch Zusatz von konzentrierten wäßrigen Alkalilösungen durchgeführt, wonach das Reaktionsprodukt mit einem organischen Lösungsmittel extrahiert wird.

GB-A-2 145 076 betrifft ein Verfahren zur Herstellung von 1,2-Alkandiolen. Dazu werden 1,2-Olefine mit Wasserstoffperoxid und Ameisensäure umgesetzt und die erhaltenen Ester mit 25%iger Natriumhydroxidlösung verseift, worauf die organische Phase destilliert wird.

Bei den vorstehenden Verfahren wird die Verseifung beziehungsweise Spaltung der entstandenen Ameisensäureester mit Hilfe von Laugen durchgeführt. Anschließend werden die erhaltenen Diole durch Extraktion oder Destillation abgetrennt. Zum einen entstehen in den Verfahren Salze der Ameisensäure, die abgetrennt und aufwendig entsorgt oder aufgearbeitet werden müssen, zum anderen ist die Abtrennung der vicinalen Diole aus dem Reaktionsgemisch aufwendig.

Aufgabe der vorliegenden Erfindung ist die Bereitstellung von Verfahren zur Herstellung von vicinalen Diolen oder Polyolen, bei denen keine Salze der Ameisensäure anfallen. Zudem sollen die Verfahren einfach durchführbar sein und in hoher Ausbeute zu den gewünschten Produkten führen.

Die Aufgabe wird erfindungsgemäß gelöst durch ein Verfahren zur Herstellung von vicinalen Diolen oder Polyolen aus einem Ameisensäureester von vicinalen Diolen oder Polyolen enthaltenden Reaktionsgemisch, bei dem das Reaktionsgemisch mit Methanol in, bezogen auf die Formylgruppen, mindestens stöchiometrischer Menge versetzt und anschließend thermisch behandelt wird, wobei Ameisensäuremethylester, Methanol und Wasser als Azeotrop ausgetragen werden und vicinale Diole oder Polyole zurückbleiben.

Die Aufgabe wird gelöst durch ein Verfahren zur Herstellung von vicinalen Diolen oder Polyolen durch Umsetzung von unsubstituierten Olefinen mit Wasserstoffperoxid und Ameisensäure zu einem Ameisensäureester enthaltenden Reaktionsgemisch, und Spaltung der Ameisensäureester, bei dem die Spaltung nach dem vorstehenden Verfahren erfolgt und die Aufarbeitung unter Zusatz katalytischer Mengen starker Säuren erfolgt.

Erfindungsgemäß wurde gefunden, daß in einem Verfahren zur Herstellung von vicinalen Diolen oder Polyolen die Spaltung der erhaltenen Ameisensäureester umgangen werden kann. Die erfindungsgemäßen Verfahren führen dabei in kurzen Reaktionszeiten zu hohen Umsätzen, hohen Ausbeuten und reinen Produkten.

Die erfindungsgemäßen Verfahren weisen insbesondere die folgenden Vorzüge auf:
- quantitativer Umsatz
- kurze Reaktionszeiten
- Produkte von hoher Reinheit werden mit hoher Selektivität erhalten
- keine Isolierung der Epoxid-Zwischenverbindung notwendig
- kein Salzanfall
- keine (Schwer)metallkatalyse
- keine organischen Lösungsmittel
- vollständige Rückführung der Ameisensäure beziehungsweise des Ameisensäure-Wasser-Azeotrops beziehungsweise des Ameisensäuremethylesters
- Verwendung von wäßrigem Wasserstoffperoxid als Oxidationsmittel
- keine zu entsorgenden Stoffe außer anfallendem Wasser.

Zunächst wird die Umsetzung von vicinalen Diolen mit Wasserstoffperoxid und Ameisensäure beschrieben.

Als Olefine können in den erfindungsgemäßen Verfahren alle Olefine eingesetzt werden, die mit Wasserstoffperoxid und Ameisensäure umgesetzt werden können. Vorzugsweise weisen die Olefine 4 bis 30+, besonders bevorzugt 6 bis 24, insbesondere 10 bis 18 Kohlenstoffatome auf. Dabei kann es sich um lineare oder verzweigte Olefine handeln. Vorzugsweise sind die eingesetzten Olefine linear.

In den Olefinen können eine oder mehrere Kohlenstoff-Kohlenstoff-Doppelbindungen vorliegen. Vorzugsweise liegt eine Kohlenstoff-Kohlenstoff-Doppelbindung vor, die endständig oder innenständig sein kann. Vorzugsweise liegt eine Doppelbindung vor, die endständig ist. Dabei ist das Olefin vorzugsweise linear.

Beispiele geeigneter Olefine sind 1-Hexen, 1-Dodecen, 1-Tetradecen, C₂₀₋₂₄-α-Olefin, C_{12/14}-α-Olefin, das insbesondere zu etwa 2/3 aus 1-Dodecen und zu 1/3 aus 1-Tetradecen besteht.

Auch Gemische von Olefinen und aus der technischen Synthese der Olefine stammende Rohprodukte können erfindungsgemäß eingesetzt werden.

Die Ameisensäure wird in der Regel als wäßrige Lösung eingesetzt. Dabei handelt es sich vorzugsweise um eine konzentrierte wäßrige Lösung mit einem Gehalt von 50 bis 100 Gew.-% Ameisensäure. Insbesondere werden reine Ameisensäure oder ein Azeotrop aus Ameisensäure und Wasser mit einem Anteil von etwa 77 Gew.-% Ameisensäure eingesetzt.

Die Konzentration des eingesetzten Wasserstoffperoxids kann in weiten Bereichen variiert werden. Vorzugsweise wird das Wasserstoffperoxid in einer Konzentration von mindestens 30%, besonders bevorzugt von mindestens 50% eingesetzt.

Das Molverhältnis von Olefin zu Ameisensäure zu Wasserstoffperoxid beträgt bei der Umsetzung 1 : 0,5 bis 5 : 1 bis 2. Besonders gute Ergebnisse werden bei Molverhältnissen von 1 : 0,5 bis 3 : 1 bis 2 erhalten. Beispiele bevorzugter Molverhältnisse sind 1 : 1 : 1, 1 : 2 : 1, 1 : 0,5 : 1 bis 2, 1 : 3,5 : 2, 1 : 2 : 2, 1 : 2 : 1,5.

Je höher der Anteil an Ameisensäure bei der Umsetzung ist, desto größer ist der Gehalt an Formylestern im erhaltenen Reaktionsgemisch.

Die Umsetzung wird vorzugsweise bei einer Temperatur im Bereich von 40 bis 100°C, besonders bevorzugt 80 bis 100°C bei Normaldruck durchgeführt. Eine Durchführung der Reaktion unter Druck ist möglich. Die Reaktionszeit beträgt in der Regel 1 bis 5 Stunden, vorzugsweise 2 bis 4 Stunden. Dabei kann die Umsetzung kontinuierlich oder diskontinuierlich durchgeführt werden. Vorzugsweise wird die Umsetzung derart durchgeführt, daß das umzusetzende Olefin mit der gewählten Menge an Ameisensäure vorgelegt wird und nach Erwärmen auf die Reaktionstemperatur mit dem Wasserstoffperoxid kontinuierlich versetzt wird. Die Zugabe des Wasserstoffperoxid erfolgt dabei vorzugsweise mit einer Rate von 0,1 bis 10 ml/min, besonders bevorzugt 0,1 bis 4 ml/min, insbesondere 1 bis 2 ml/min, bezogen auf 50%ige H₂O₂ und 1 Mol Olefin.

Die Umsetzung wird in Gegenwart katalytischer Mengen einer starken Säure, wie Schwefelsäure oder Flußsäure durchgeführt.

Die Umsetzung wird in der Regel in Abwesenheit eines Lösungsmittels - abgesehen vom im Reaktionsgemisch vorliegenden Wasser - durchgeführt. Somit muß auch kein organisches Lösungsmittel aus dem Reaktionsgemisch entfernt werden.

Zur Optimierung der Umsetzung kann während der Reaktion ein Teil der wäßrigen Phase durch konzentrierte Ameisensäure ersetzt werden. Ein kontinuierliches Verfahren zur Konzentrierung der wäßrigen Phase ist möglich.

Nach der Umsetzung wird in der Regel ein Reaktionsgemisch erhalten, das sich in eine organische Phase und eine wäßrige Phase trennen läßt. Die wäßrige Phase kann zur Konzentrierung der Ameisensäure beispielsweise durch Destillation aufgearbeitet werden, so daß ein Ameisensäure-Wasser-Azeotrop erhalten wird, das in die Umsetzung zurückgeführt werden kann.

Das Reaktionsgemisch kann wie folgt aufgearbeitet werden:

Werden Olefine, die mindestens 10 C-Atome aufweisen, zur Umsetzung mit Wasserstoffperoxid und Ameisensäure eingesetzt, so wird nach der Umsetzung ein Reaktionsgemisch erhalten, das einen hohen Anteil an vicinalen Diolen und einen relativen geringen Anteil an Ameisensäureestern aufweist. Vorzugsweise erfolgt hierbei die Umsetzung mit einem Molverhältnis von Olefin zu Ameisensäure zu Wasserstoffperoxid von 1 : 1, 7 : 1,7.

Erfindungsgemäß wird ein Reaktionsgemisch, das durch Umsetzung von Olefinen mit Ameisensäure und Wasserstoffperoxid erhalten wird, mit Methanol versetzt. Dabei ist die Menge an Methanol, bezogen auf die im Reaktionsgemisch vorliegenden Formylgruppen (in freier Ameisensäure oder in Estergruppen) mindestens stöchiometrisch. Vorzugsweise beträgt das Molverhältnis von Methanol zu Formylgruppen 50 : 1 bis 1 : 1, besonders bevorzugt 30 : 1 bis 5 : 1.

Nach Versetzen des Reaktionsgemisches mit Methanol wird dieses thermisch behandelt, wobei die vorliegenden Ameisensäureester der vicinalen Diole mit Methanol umgeestert werden und freie Ameisensäure in den Methylester überführt wird. Sodann werden gebildeter Ameisensäuremethylester und gegebenenfalls vorliegendes überschüssiges Methanol und Wasser als Dampfphase als Azeotrop ausgetragen. (Der Siedepunkt von Ameisensäuremethylester beträgt unter Normaldruck 32°C, von Methanol 65°C). Dazu wird das Reaktionsgemisch vorzugsweise unter Rückfluß erhitzt. Es kann auch unter Druck gearbeitet werden. Die thermische Behandlung beziehungsweise das Erhitzen zur Umsetzung mit dem Methanol und zur Entfernung des gebildeten Ameisensäuremethylesters kann auch in einem Schritt, das heißt gleichzeitig erfolgen. Die Umsetzung kann dabei in Art einer Reaktivdestillation in einer geeigneten Vorrichtung durchgeführt werden.

Der entstandene Ameisensäuremethylester kann direkt in die Ameisensäuresynthese zurückgeführt werden, die über die Stufe des Ameisensäuremethylesters erfolgt. Somit kann die Ameisensäure in einfacher Weise in bereits bestehenden Anlagen zurückgewonnen und in die Umsetzung zurückgeführt werden.

Die Methanolyse verläuft für Ameisensäureester von vicinalen Diolen aller vorstehend aufgeführten Olefine schnell und quantitativ. Das Verfahren ist somit universell einsetzbar. Zudem weist es den Vorteil auf, daß, insbesondere, wenn keine Abtrennung der wäßrigen Phase vor der Methanolyse stattfindet, keine Ameisensäure destillativ entfernt wird. Ameisensäure, beziehungsweise ein Ameisensäure-Wasser-Gemisch ist korrosiv, so daß die für die Entfernung eingesetzte Apparatur aus korrosionsbeständigen Materialien aufgebaut sein muß.

Zudem weist diese Verfahrensvariante den Vorteil auf, daß die Methanolyse deutlich schneller als die Hydrolyse abläuft. Es werden wiederum katalytische Mengen starker Säuren, wie Schwefelsäure oder Flußsäure zugesetzt werden, um die Umsetzung nochmals zu beschleunigen.

Bei der erfindungsgemäßen Herstellung der vicinalen Diole erfolgt die Umsetzung der Olefine vorzugsweise vollständig, damit im Reaktionsgemisch nach der Umsetzung keine Olefine verbleiben.

Die Aufarbeitung der erhaltenen Ameisensäureester von vicinalen Diolen oder der vicinalen Diole erfolgt ohne Zusatz von Basen und ohne Vorliegen von alkalischen Bedingungen, bei einem pH-Wert kleiner 7.

Nachstehend wird die Erfindung anhand von Beispielen näher erläutert.

### Beispiel 1

### Herstellung von Formylestern (Molverhältnis Olefin/Ameisensäure 1 : 2)

In einem Laborreaktor wurden 95,3 g (0,5 Mol) C_{12/14}-α-Olefin (bestehend zu etwa 2/3 aus 1-Dodecen und zu etwa 1/3 aus 1-Tetradecen) und 46,0 g (1,0 Mol) Ameisensäure vorgelegt. Man erwärmte diese Mischung auf eine Temperatur von 100°C und tropfte anschließend über einen Zeitraum von 5 bis 6 Stunden 68,0 g (1,0 Mol) Wasserstoffperoxid als 50%ige wäßrige Lösung hinzu (0,2 g/min). Anschließend rührte man bei 100°C noch etwa 10 Minuten nach, bis keine Doppelbindungen mehr nachweisbar waren (bestimmt über die Iodzahl).

Die organische Phase wies folgende Analysewerte auf:
Organische Phase (145,4 g); Säurezahl 1,632 mmol/g, Wassergehalt: 14,0%, organische Komponenten: 28,2% Formylester und 71,8% 1,2-Diole.
Wäßrige Phase (23,2 g): Säurezahl 5,230 mmol/g (überschüssige Ameisensäure).
Peroxid war nicht mehr nachweisbar.

### Beispiel 2

### Synthese von Formylestern (Molverhältnis Olefin/Ameisensäure 1 : 3,5)

In einem Laborreaktor wurden 95,3 g (0,5 mol) C_{12/14}-α-Olefin und 80,5 g (1,75 mol) Ameisensäure vorgelegt. Man erwärmte diese Mischung auf eine Temperatur von 100°C und tropfte anschließend über einen Zeitraum von 105 Minuten 68,0 g (1,0 Mol) Wasserstoffperoxid als 50%ige wäßrige Lösung zu (0,65 g/min). Anschließend rührte man bei 100°C noch etwa 40 Minuten nach, bis keine Doppelbindungen mehr nachweisbar waren (bestimmt über die Iodzahl).

Peroxid war nur noch in Spuren (0,013 mmol/g) nachweisbar.

### Beispiel 3

In einem Laborreaktor wurden 189,2 g (1,0 mol) C_{12/14}-α-Olefin (C_{12/14}-α-Olefin besteht zu etwa 2/3 aus 1-Dodecen und zu 1/3 aus 1-Tetradecen) und 73,6 g (1,6 mol) Ameisensäure vorgelegt. Man erwärmte diese Mischung auf 100°C und tropfte anschließend über einen Zeitraum von 2 h 115,7 g (1,7 mol) Wasserstoffperoxid (50%ig), das heißt ca. 1 g/min, hinzu. Anschließend rührte man bei 100°C noch etwa 30 min nach, bis keine Doppelbindung (Iodzahl) und kein Peroxid mehr nachweisbar waren.

Man versetzte mit 64,1 g (2,0 mol) Methanol und 0,1 g konzentrierte Schwefelsäure und erhitzte 30 min unter Rückfluß und destillierte anschließend bei Normaldruck den Ameisensäuremethylester, Methanol und Wasser ab.

### Analytik

Ausbeute: quantitativ 1,2-Dodecandiol und -1,2-Tetradecandiol
Säurezahl: 0,011 mmol/g, Wassergehalt: 0,3%.

## Patentansprüche

1. Verfahren zur Herstellung von vicinalen Diolen oder Polyolen durch Umsetzung von unsubstituierten Olefinen mit Wasserstoffperoxid und Ameisensäure zu einem Ameisensäureester enthaltenden Reaktionsgemisch und Spaltung der Ameisensäureester, **dadurch gekennzeichnet, dass** die Spaltung erfolgt, in dem das erhaltene Reaktionsgemisch mit Methanol in, bezogen auf die Formylgruppen, mindestens stöchiometrischer Menge versetzt und anschließend thermisch behandelt wird, wobei Ameisensäuremethylester, Methanol und Wasser als Azeotrop ausgetragen werden und vicinale Diole oder Polyole zurückbleiben, wobei das Molverhältnis von Olefin zu Ameisensäure zu Wasserstoffperoxid 1 : 0,5 bis 5 : 1 bis 2 beträgt und die Aufarbeitung unter Zusatz katalytischer Mengen starker Säuren erfolgt.

2. Verfahren nach Anspruch 1, **dadurch gekennzeichnet, dass** die Olefine C₄₋₃₀₊-Olefine sind.

3. Verfahren nach Anspruch 1 oder 2, **dadurch gekennzeichnet, dass** die thermische Behandlung in einem Dünnfilmverdampfer erfolgt.

4. Verfahren nach einem der Ansprüche 1 bis 3, **dadurch gekennzeichnet, dass** das Molverhältnis 1 : 0,5 bis 3 : 1 bis 2 beträgt.

## Claims

1. A process for preparing vicinal diols or polyols by reacting unsubstituted olefins with hydrogenperoxide and formic acid to form a formic ester containing reaction mixture and cleaving of the formic acid ester, wherein the cleaving occurs in that methanol is added, in an amount which is at least stoichiometric relative to the formyl groups, to the resultant reaction mixture, which is subsequently thermally treated with removal of methyl formate, methanol and water as azeotrope, and vicinal diols or polyols remaining behind wherein the molar ratio of olefin to formic acid to hydrogenperoxide is 1:0.5 to 5:1 to 2 and the work up occurs under addition of catalytic amounts of strong acids.

2. A process as claimed in claim 1, **characterized in that** the olefins are C₄-₃₀₊ olefins.

3. A process as claimed in claim 1 or 2, **characterized in that** the thermal treatment takes place in a thin-film evaporator

4. A process as claimed in any one of the claims 1 to 3, **characterized in that** the molar ratio is 1:0.5 to 3:1 to 2.

## Revendications

1. Procédé de fabrication de diols ou de polyols vicinaux par transformation d'oléfines non-substitués avec peroxyde d'hydrogène et acide formique dans une mélange réactif contenant le formiate, et division du formiate, **caractérisée en ce que** le mélange réactif obtenu avec le méthanol, au minimum en quantité stoechiométrique, selon les groups de formyl, est transformé et finalement traité thermiquement, moyennant quoi le formiate, le méthanol et l'eau sont distribués en tant que mélange azéotrope, et des diols ou des polyols vicinaux demeurent, tout en le rapport molaire de l'oléfine en formiate en peroxyde d'hydrogène est de 1:0.5 à 5:1 à 2 et le remise à neuf résulte en suppléments de quantité catalytique de l'acide intense.

2. Procédé selon la revendication 1, **caractérisé en ce que** les oléfins ont les oléfines C₄₋₃₀₊.

3. Procédé selon la revendication 1 ou 2, **caractérisé en ce que** le traitement thermique a lieu dans un évaporateur à film fin.

4. Procédé selon les revendications 1 de 3, **caractérisé en ce que** le rapport molaire est de 1:0.5 à 3:1 à 2.
